# EUROPEAN PATENT SPECIFICATION

(11) **EP 2 648 786 B1**
(45) Date of publication and mention of the grant of the patent: **12.05.2021**
(21) Application number: 11760538.6
(22) Date of filing: 19.09.2011
(51) Int. Cl.: A61M 15/00, B65B 9/04, G01F 11/02, B65B 1/36, G01F 11/24, A61J 3/07, B65B 1/38

(54) **APPARATUS FOR DISPENSING POWDER**
VORRICHTUNG ZUR ABGABE EINES PULVERS
APPAREIL POUR DISTRIBUTION DE POUDRE

(30) Priority: 06.12.2010 GB 201020646
(43) Date of publication of application: 16.10.2013
(73) Proprietor: MPAC LAMBERT LIMITED, Station Road Tadcaster North Yorkshire LS24 9SG (GB)
(72) Inventor: BAKER, Jeremy, Coventry CV4 8HS (GB); SHIRLEY, Graham, Coventry CV4 8HS (GB); HAYTO, Ian, Coventry CV4 8HS (GB); MAYES, Geoffrey, Coventry CV4 8HS (GB)
(74) Representative: Williams, Michael Ian
(86) International application number: PCT/GB2011/051753
(87) International publication number: WO 2012/076859

(56) References cited:
- DE-A1- 2 262 362
- GB-A- 1 309 424
- US-A- 2 799 432
- US-A- 2 888 963
- US-A- 3 446 404
- US-A- 3 656 518
- US-A- 4 671 430
- US-A1- 2003 126 831
- US-A1- 2006 260 274
- US-A1- 2010 044 398
- US-B2- 7 661 248

## Description

This invention relates to apparatus for dispensing defined amounts of a powder material such as a pharmaceutical powder for use as a dry powder inhaler.

Apparatus of this type is used to dispense powder into an inhaler device such as blister packs and many different types of such apparatus exist in the art. The present invention is concerned with a development of this type of apparatus.

A number of known systems include drums for delivering powder from a hopper to a receptacle. For example, the documents US 2,913,018 and US 3,656,518 describe arrangements in which powder is drawn into a chamber in a drum using negative gas pressure. In both of these arrangements the powder is expelled using a positive gas pressure. These arrangements are considered disadvantageous because the use of pressurised gas can cause an undesirable dusting of powder on surfaces of the apparatus and the receptacle.

Another known arrangement is described in GB 999,030. In this arrangement powder can be transferred from a hopper to receptacles in charge chambers of a drum. No pressurised air is used to retain the powder in the receptacles. The powder is retained by an annular housing as it rotates, but the arrangement may cause powder to be lost from the receptacles as the drum is rotated. In GB 999,030 powder is dispensed from the charge chambers using a spring loaded ejector which is arranged radially within the circular drum.

The document US 3,446,404 describes a rotary feed roll for filling capsules with powder from a hopper. The roll is covered in a rubber sleeve which can be deformed using negative air pressure to form charged chambers. Powder can be dispensed from the charged chambers by applying a positive air pressure, and making use of the elasticity of the rubber sleeve.

US 2006/260274 discloses a dosing device for feeding an infusion product. It has a rotary drum positioned between a web of filter material and a hopper for containing the infusion product. The drum has a plurality of radial cells in which there is a slide piston type dosing means. Each dosing piston is driven axially by eccentric cam actuating means as the rotary drum rotates. Each piston has a crank mechanism designed to act coaxially on the piston to cause the piston to move in a direction that is perfectly aligned with a longitudinal axis of the respective dosing cell.

US 2888963 discloses a carton filling apparatus which is capable of introducing a measured quantity of product from a hopper to a carton or other receptacle. It discloses a rotary filling apparatus with a series of pockets having plungers inside by means of which the product may be expelled by means of which the size of the pockets may be varied. The plungers are controlled to present a pocket of adjusted depth to receive the goods flowing into the pockets. At the completion of the pocket filling operation the plungers retract in a manner to enlarge the volume of the pocket. As a result the material within the pocket is loosely contained and crowding of the material within the pockets against the peripheral pocket enclosing more is avoided.

US 2003/126831 discloses a wheel which has a plurality of moulds disposed at angular intervals and which extend from the cylindrical peripheral surface of the wheel radially towards the centre axis. Each of the moulds contains a piston which is movable back and forth between a retracted position for filling the mould with a substance, and an advanced position for ejecting the consolidated formed body in a radially outward direction onto packaging material which is arranged horizontally below the wheel.

US 2010/044398 discloses a dosing device for dosing small particulate pharmaceutical preparations. It discloses a chamber for receiving a larger amount of the preparation and a dosing unit comprising a rotary valve carrying at least one dosing chamber for receiving a defined partial amount of the preparation whereby the dosing chamber is movable between a filling position, in which it is to be filled from the chamber, and a dispensing position in which the preparation is to be dispensed from the dosing chamber. The rotary valve is rotatable at a substantially horizontal access and has an adjustment device for setting the volume in the dosing chamber.

US 2799432 discloses a depositing machine which is arranged to deposit either increments of a single selected granular or liquid material such as salt or simultaneously increments of two or more such materials. This document discloses a revolving drum assembly which may have one or more compartments and a stationary hopper assembly with a corresponding number of compartments and transfer chutes. The drum and hopper assemblies are mounted so that the drum may rotate and so that the drums discharge valves may be actuated by a cam unit mounted on the base. Gravity and centrifugal force continue to effectively discharge material from the discharge valves into an adjustably mounted funnel.

GB 1309424 discloses apparatus for measuring and dispensing predetermined amounts of powdered material. The apparatus has a measuring chamber with an open end and a piston inside the chamber. The piston is arranged to be pervious to a gaseous medium and impervious to the powdered material to be dispensed. During dosing the measuring chamber is subjected to negative pressure through the piston head to introduce a predetermined measured amount of powdered material into the chamber. The piston is then moved to eject the powdered material and the apparatus includes a step of compacting the powdered material before or after charging the measuring chamber. The slug of compacted powder may be blown clear of the piston head by applying positive gas pressure to the piston head.

US 7,661,248 discloses a device for dosing and forming pods for products for infusion, such as coffee and tea. The device includes a dosing station comprising a fixed hopper mounted to face a revolving drum. The hopper has an arc-shaped discharge portion to peripherally follow a passing surface of the drum in such manner that the product is dosed in a predetermined area. The revolving drum is equipped with a plurality of pistons arranged radially on the surface of the drum, each piston having a hollow head defining an impression for receiving a dose of the product fed by the hopper. Each of the pistons performs a series of synchronised movements in a radial direction by virtue of a drive mechanism comprising a cam and a cam follower.

US 4,671,430 discloses apparatus for apportioning powdered material into each of a series of containers. The apparatus includes a powdered material hopper having a dispensing opening and a rotatable filling head contiguous to the dispensing opening. The rotatable filling head includes a plurality of radially situated chambers each having an open outer end for displacement between the material hopper and the series of containers. A pneumatic source is provided for assisting the filling and emptying of the chambers during the apportioning process. Within each chamber, the volume of powdered material received is defined by a piston having a porous surface. The piston engages a threaded stem longitudinally fixed in position yet rotatable about its longitudinal axis to effect displacement of the piston head and support longitudinally within the chamber. A seal is provided at the radially inner end of the threaded stem to ensure positive pneumatic action through the porous piston surface.

DE 22 62 362 discloses a device for dosing and packaging loose or pourable goods, having at least one first element, which has at least one space of a predetermined volume, and at least one second element, which has a first opening for the passage of the loose or pourable goods. The first element consists of a hollow cylinder or a drum whose cylindrical outer casing has a plurality of openings. In the region of the edge of each opening, the edge of a corresponding cup element is welded, which extends radially to the centre of the cylinder and has a predetermined volume. A piston runs inside this cup element. A piston rod is guided in a corresponding bore, which is provided in the bottom of the cup element. The piston rod is engaged with a cam fixedly arranged inside the hollow cylinder. A hopper is provided above the cylinder and a discharge funnel is provided below the cylinder.

One object of the present invention is to provide an apparatus that can avoid unintentional loss of powder from a drum assembly without causing an undesirable dusting of surfaces.

Another object of the invention is to provide an apparatus with a reduced drum diameter that can also be cleaned more easily.

According to the invention there is provided an apparatus for dispensing a predetermined dose of powder as defined in claim 1.

In this way a powder dispenser can be provided with a reduced diameter in comparison to a drum assembly having a radial piston cylinder or a radial piston spring. A reduced diameter is advantageous because it decreases the overall cost of the apparatus.

By providing the piston in a position that is axially displaced from the driving assembly it is also possible to isolate the driving assembly from the powder filling region. This is advantageous because it means that powder is less likely to interfere with moving parts in the driving assembly. Also, the drum assembly, including the piston, can be easily disconnected from the driving assembly for cleaning. This means that powder coated surfaces can be cleaned easily without stripping the entire machine apart. In addition, the driving assembly can be isolated from any cleaning products which could interfere with its normal operation.

Preferably the apparatus includes a plurality of pistons, each associated with a separate aperture. In this way the drum may be able to fill a plurality of powder receptacles simultaneously.

The alignment adjuster may be for adjusting the inclination of the or each piston relative to its associated aperture. It is important that each piston can slide easily into an aperture with minimal clearance around the periphery of the piston. This minimises any loss of powder during operation of the apparatus. The alignment adjuster is preferably arranged to provide microscopic adjustments in the inclination of the piston in order to optimise alignment.

A plurality of pistons may be provided on the flexible piston plate in the drum assembly. The piston plate may be flexed by the alignment adjuster so that all of the pistons on the piston plate can have their alignment adjusted at the same time.

The radial movement of the or each piston may be provided by the geared-down motion of a component in the drive assembly. Thus, a large motion in the drive assembly can be converted into a smaller movement in the piston. For example, a stroke reducer may be provided in the drive assembly for converting an axial motion into a radial motion with a ratio of approximately 10:1. The stroke reducer can increase the accuracy with which the piston is positioned, thereby improving accuracy in the control of the powder dosage.

The position of the piston may be controlled in order to adjust the volume of the compartment. By adjusting the position of the piston it is possible to adjust the position of the base of the compartment and thereby alter the volume of the compartment. In this way it is possible to adjust the dose that is delivered by the apparatus.

The piston may be arranged to compress the powder in the compartment. In this way, powder can be received in the compartment in the receiving position, and the powder can be subsequently compressed by the piston. The motion of the piston can be carefully controlled to maintain the integrity of the dose and to allow any air to escape.

The apparatus may comprise a compaction band against which the piston can compress the powder. Preferably the compaction band is arranged to follow the motion of the compartment, at least in part, while it moves from the receiving position to the dispensing position. The compaction band can, therefore, lie against the top of the compartment while the piston compresses the powder. Preferably there is no relative circumferential movement between the compaction band and the compartment when the piston compresses the powder; this minimises any loss of powder between the compartment and the compaction band.

The drum assembly, the piston and the driving assembly may be mounted on a carriage that permits translation of the components. By mounting the apparatus on a translatable carriage powder can be delivered into receptacles on a moving conveyor belt with a minimal relative movement between the drum compartment and the receptacles. This arrangement allows powder to be dispensed more slowly which can reduce powder loss and improve dose integrity.

Preferably the drum assembly can be removed quickly and easily so that it can be cleaned. The drum assembly can be removed without a complicated sequence of steps for stripping the machine down because the drum assembly is cantilevered relative to the drive assembly, which means the drum assembly is connected to the drive assembly from one side only.

A predetermined quantity of powder may be accumulated in the compartment and delivered at the dispensing position. By closing the compartment with a foil the apparatus can deliver powder to the dispensing position without any loss of powder. This can be achieved without the use of pressurised air in order to prevent powder from coating surfaces of the apparatus.

The foil or cover portion is preferably designed to cover the aperture in the support portion and thereby close the compartment. When the compartment is closed it may be safe to move the drum assembly from the filling position to the dispensing position without any loss of powder.

The drum assembly may include both the foil and the support portion and preferably both of these components are arranged to rotate together. Thus, the foil can close the compartment and rotate together with the powder until complete alignment is attained with the receiver. Accordingly there is no possibility for any loss of powder due to failure of inter-particle frictional forces to retain the unsupported powder at an end of the compartment during the final alignment.

Preferably the drum assembly has a cylindrical portion mounted for movement relative to the support portion, said cylindrical portion having said foil on its outer surface.

Preferably the compartment is filled completely with powder so that there is a predetermined dosage of powder. The edge of the foil may doctor off any excess powder when it closes the compartment.

Preferably the drum assembly is rotatable between the filling and dispensing positions. Preferably still the drum assembly is rotatable through 180°, with the filling position at '12 o'clock' and the dispensing position at '6 o'clock'.

Preferably the foil has a plurality of spaced through apertures and the support portion has a corresponding number of spaced apertures. In this way a number of compartments can be filled with powder in a single motion. This can provide a convenient mechanism for filling a number of receptacles, such as blister packs, quickly. The apertures may be spaced linearly and regularly.

The or each aperture of the support portion may have an associated piston which can be moved into the inner end of the aperture. The piston may be arranged to dispense the contents of the compartment quickly in the dispensing position. In addition the piston may define the base of the compartment so that the initial position of the piston defines the volume of the compartment at the filling position.

Each piston may be carried on a carrier, and the carrier may be cam operated to effect movement of said piston or pistons.

Preferably said foil and said support portion are moveable axially relative to another to cause opening or closing of said compartment. However, a number of alternative types of relative movement would be possible in order for the foil to close the compartment. For example, the foil may move circumferentially relative to the support portion.

A receptacle or receptacles may be located adjacent the dispensing position to receive dispensed powder. Non-limiting examples of the receptacle include a blister strip formed pocket, a bottle, and the bottom portion of a capsule.

The apparatus may include a hopper containing powder for delivering to the or each compartment. In addition the hopper may include means for compressing the powder towards the or each compartment, such as a roller. An agitator may be provided for aerating the powder in the hopper before it is compressed towards a compartment.

The apparatus may include a cleaning station positioned between the dispensing position and the filling position. The cleaning station may use pressurised air to remove small amounts of powder from an empty compartment in the support portion, including the faces of the pistons.

The or each piston may be arranged to compact the powder in a closed compartment. Thus, the powder may be compacted between the piston and the foil that closes the end of the compartment. This may be desirable so that the piston can lightly compress the dose to conform to the required properties of the powder to be dispensed.

The apparatus may be mounted on a carriage that permits translational movement relative to a receptacle. In this way the drum may be translated in synchronisation with a moving receptacle in order to optimise the filling of the receptacle.

The powder may be pharmaceutical powder for use as a dry powder inhaler. However, the apparatus may be used for dispensing predetermined quantities of a number of different types of powder.

Preferred features of the invention are set out in the dependent claims.

The invention will be described now by way of example only with particular reference to the accompanying drawings. In the drawings:
Figure 1 is schematic illustration of a device
Figure 2 is an exploded view of the device of Figure 1;
Figure 3 is a view showing part of the device of Figure 1;
Figure 4 is a sectional view showing another part of the device of Figure 1;
Figures 5, 6 and 7 illustrate the operation of the device of Figure 1;
Figure 8 is a perspective view of a detailed embodiment of a device
Figure 9 is a plan view of the device of Figure 8;
Figure 10 is a sectional view along the line 9-9 of Figure 9;
Figure 11 shows the clamping of the foil to the drum;
Figure 12 shows an alternative form of the foil structure;
Figure 13 shows a schematic illustration of a device
Figure 14 is a cross-sectional view of a powder dispensing apparatus
Figure 15 is a more detailed view of one of the features in Figure 14.

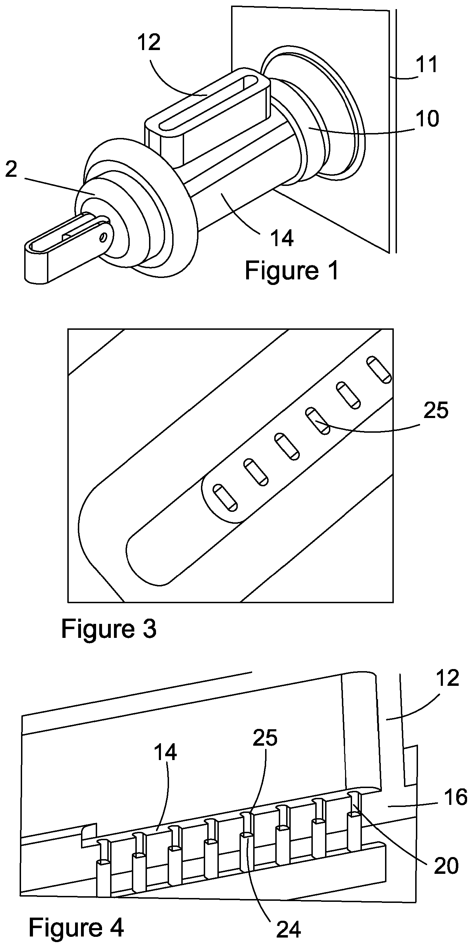

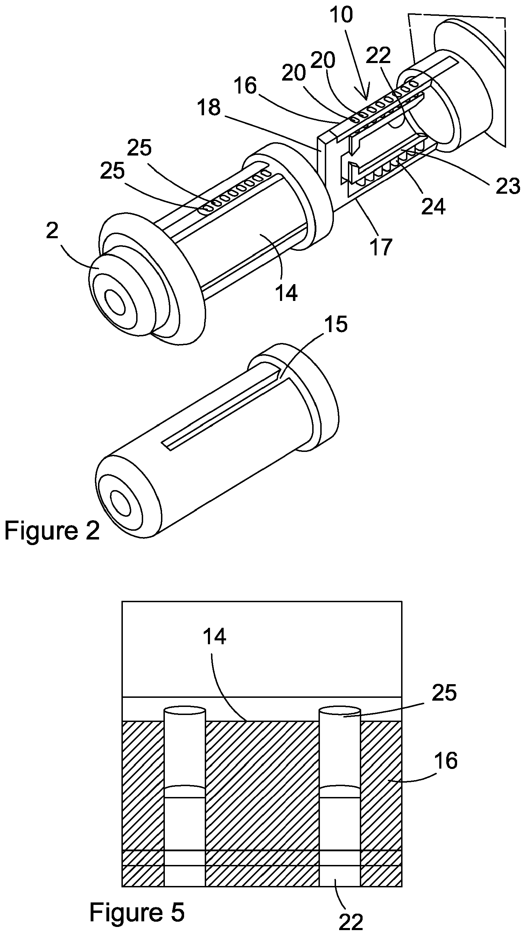

Referring to Figures 1 to 7 a device for dispensing defined amounts of a powder comprises a drum 2 which is supported on a structure 10 extending outwardly from a wall 11 of a housing. A hopper 12 is mounted above the drum 2. A thin stainless steel foil 14 is wrapped around the circumference of the drum 2. Referring to Figure 2 of the drawings the drum 2 has a longitudinal slot 15 that is arranged to coincide with the location of the hopper 12. The support structure 10 extending out from the housing 11 has upper and lower limbs 16 and 17 which project outwardly from the wall 11. The free ends of the limbs 16 and 17 are connected by a member 18. Each limb has formed therein a series of longitudinally spaced through apertures or bores 20. Mounted between the limbs 16 and 17 are longitudinal members 22 and 23 each of which carries a series of outwardly extending pins 24, the number and spacing of the pins corresponding to the number and spacing of the apertures 20. The longitudinal members 22 and 23 are moveable radially by a mechanism not shown in Figure 2 so that the pins act as pistons and can move into the apertures 20 to thereby close the lower end of the apertures. This enables the apertures to define a variable size cavity or compartment depending upon the extent to which the pistons move into the apertures. The capacity of each cavity is set by an adjustment screw stop which limits the extent of the inward radial pin travel to control the fillable volume of the cavity. The upper limb 16 is received within the slot 15 formed in the drum when the drum is mounted upon the structure 10 and the limb 17 is received in a similar longitudinal slot not shown in the drawings.

The foil 14 wrapped around the circumference of the drum has formed therein a series of apertures 25 whose number and spacing corresponds to the number and spacing of the apertures 20. By appropriate placement of the drum longitudinally on the support structure 10 the apertures 25 formed in the foil 14 can be aligned or misaligned with the apertures 20 to thereby open or close the cavities or compartments.

In use the drum 2 is located longitudinally relative to the limbs 16 and 17 so that the apertures 25 of the foil 14 are aligned with the apertures 20. The elements 22 and 23 are moved radially outwardly so that the pistons extend into the apertures 20 to form a cavity or compartment of a predefined size. Powder is located in the hopper 12 and enters the cavities by way of the apertures 25 in the foil 14. The powder can be forced into the cavities by an arrangement of rollers and blades not shown in the drawings. Such arrangements will be known to those skilled in the art. Figure 5 of the drawings illustrates a configuration in which the bases of the apertures 20 formed in the limbs 16 are closed by the pistons 24. The apertures 25 formed in the foil layer are shown to be in alignment with the upper side of apertures 20 formed in the element 16. In this configuration powder can enter the cavity defined by the aligned apertures.

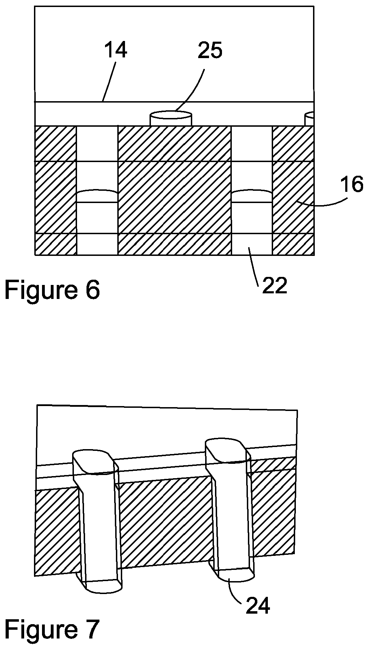

Once the cavities have been filled the drum and hence the foil 14 are displaced longitudinally relative to the limb 16 so that the apertures 25 in the foil become displaced from the apertures 20 in the limbs 16. This situation is illustrated in Figure 6 of the drawings. In this configuration the cavities or compartments containing the powder are closed.

When the compartments containing powder have been closed the elements 22 and 23 can be moved radially outwardly so that the pistons 24 can compact the powder against the foil 14. This optional compaction or densification may be arranged to lightly compress the powder so that the powder particles can be held together by cohesion and dispensed into a receptacle with predetermined compaction properties.

The drum with the structure 10 is then rotated so that the cavities move from the upper, filling position illustrated in Figure 2 of the drawings through 180° to a lower position from which the powder in the cavities can be dispensed to a receptacle which is not shown in Figures 1 to 7. When the drum is rotated the foil 14 is rotated as well. This means that there is no relative movement between the powder and the foil 14 during rotation. The optional compaction or densification can take place before, during or after rotation of the drum, but optimal efficiency may be achieved if compaction is performed during rotation.

Once the drum and associated structure have been rotated through 180° the foil 14 is then adjusted longitudinally so that the apertures 25 become aligned with the apertures 20. In order to eject the powder from its respective cavity the elements 22 and 23 are moved radially outwardly so that the pistons 24 move radially outwardly and force the powder from the cavity into the receptacle. This is shown in Figure 7. The receptacles used may be any type of receptacle for receiving dosed quantities of powder, such as, for example, a blister-type pack.

The apparatus may further include a cleaning station (not shown) for cleaning at least the compartments, the support portion 10 and the pistons 24 when they move from the dispensing position towards the filling position. Preferably the drum is arranged to dwell adjacent the cleaning station so that pressurised air can be used to expel any remnants of powder from the apparatus.

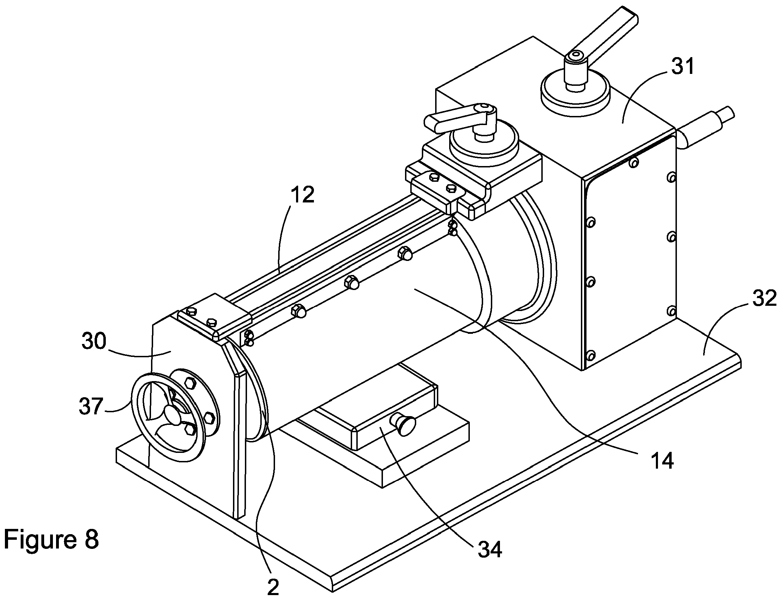

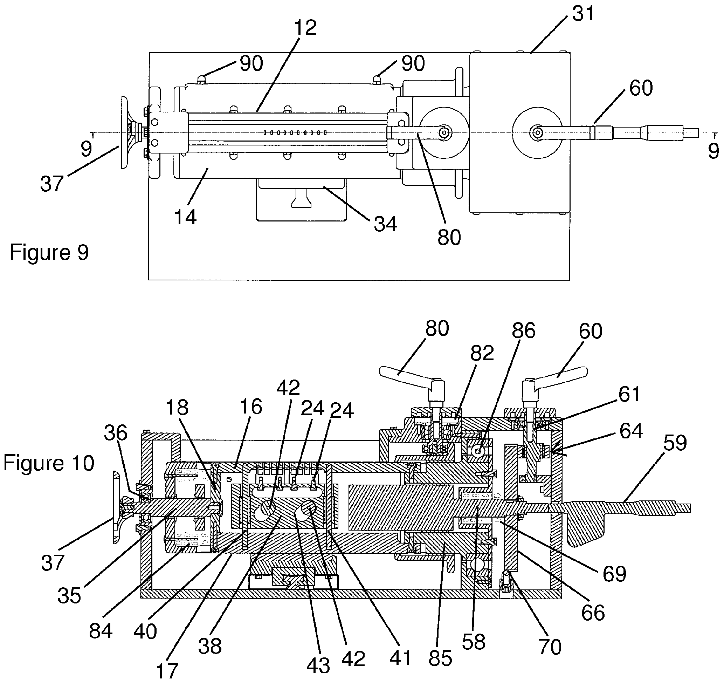

Referring to Figures 8, 9 and 10, a more detailed embodiment of a dosing device in accordance with the present invention will be described. Elements corresponding to those shown in Figures 1 to 7 are shown by like reference numerals. With reference to Figure 8 the drum with its encircling foil is shown mounted between an upstanding plate 30 and housing structure 31, each of which is mounted upon a supporting base 32. A receptacle 34 for receiving dispensed powder is mounted on the supporting base 32 beneath the drum. Referring now to Figures 8 to 10 the end wall 30 supports a shaft 35 which extends through an aperture in the wall 30. The shaft 35 is supported in the aperture on a bearing 36 so that the shaft can rotate relative to the wall. The outer end of the shaft carries a manually operable wheel 37. The internal end of the shaft 35 is connected to the member 18.

The array of pistons 24 are shown mounted upon an element 38, which is guided for radial movement by way of slides 40,41 extending between the limbs 16 and 17. The supporting element 38 is provided with cams 42 which locate in inclined slots 43. A shaft 58 extends through the housing 31 towards the mounting element 38. At the outer end of the shaft 58 a micrometer adjusting mechanism 59 is provided external to the housing 31. The micrometer 59 provides a means to set the filling volume of the cavity or cavities and thereby controls the dose weight. The shaft 58 can be moved longitudinally by means of an arrangement comprising a handle 60 which has a downwardly extending shaft 61 connected to an eccentric arrangement 64. Rotation of the handle 60 about its shaft 61 causes through the eccentric lateral movement of a disc 66 through which the shaft 68 extends. This movement occurs against the bias of a return spring 69. Movement of the disc 66 causes a corresponding longitudinal movement of the shaft 58. At its lower extremity the disc 66 is guided by a detent arrangement 70 which locates the fill and discharge rotational positions.

A second handle 80 is supported relative to the housing 31 and is connected to a second eccentric arrangement 82. Operation of the handle 80 to rotate the handle results in the eccentric causing longitudinal movement of the drum and hence its foil sleeve 14 to effect the relative longitudinal motion described with reference to Figures 1 to 7. This movement occurs against the bias of a return spring 84

The internal ends of the limbs 16 and 17 are connected to a sleeve 85, which is supported within a bearing arrangement 86 to thereby allow rotary motion of the drum with the structure of limbs the 16 and 17.

Thus it can be seen in the detailed arrangement of Figures 8 to 10 that radial movement of the pistons 24 is effected by rotation of the handle 60. Rotation of the handle 60 results in longitudinal movement of the shaft 58 and this causes the element 38 to move upwardly by virtue of sliding motion of the cams 42 within their slots 43. The amount by which the pistons are moved radially can be controlled by appropriate rotation of the handle 60 and fine adjustment can be achieved by operation of micrometer adjustment. Thus this operation sets the size of the cavity to be used in any dosing operation.

In this arrangement the radial movement of the pistons 24 is caused by rotation of the handle 60, which is located axially of the pistons, with respect to the main axis of the drum 2. This is preferable to providing radial driving means for the pistons 24, such as radial springs, because it allows the diameter of the drum 2 to be minimised.

Sliding movement of the drum 2 and its foil 14 relative to the ports 20 is achieved by operating the handle 80 to thereby open or close those ports. It will be appreciated that the cavities are filled by powder from the hopper 12 in the manner already described, with reference to Figures 1 to 7.

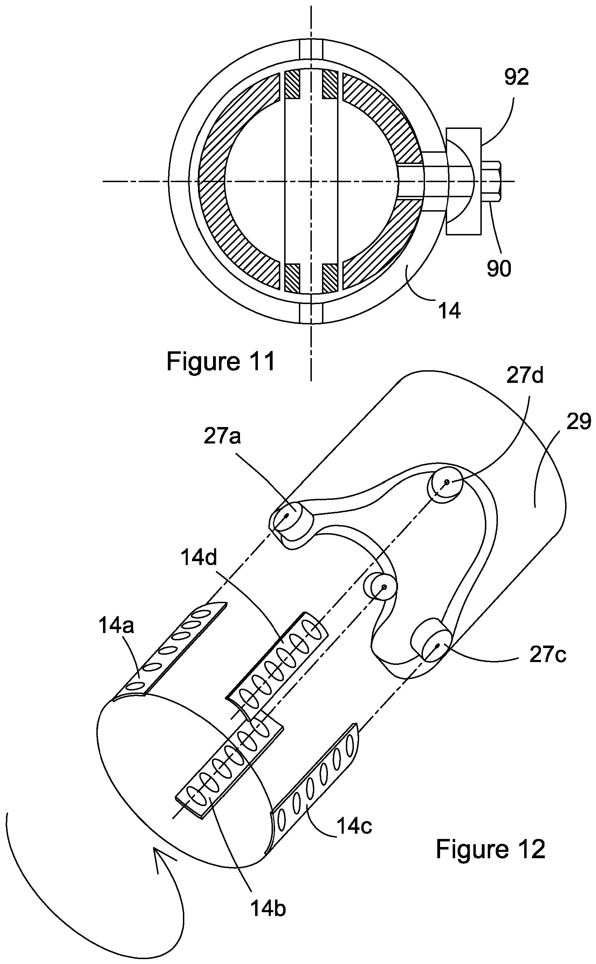

The foil 14 is secured to the drum by bolts 90 which sandwiches adjacent ends of the foil between the face of the drum and a clamp bar 92 (see Fig. 11). The foil or cover 14 which is made from stainless steel and should be as thin as practicable whilst maintaining its necessary strength. Typically the foil will have a thickness of the order of 75µm. Once the cavities have been filled and the foil has been moved to a position in which those cavities are closed, the drum together with the limbs 16 and 17 of the support system can be rotated through 180° by operation of the handle 37. This brings the cavities to a position immediately above the receiving receptacle 34 for the dosed quantities of powder. Once in this position the drum and its foil are adjusted longitudinally by operation of the handle 80 in order to open the cavities. The shaft 58 is then actuated by operation of the handle 60 in order to force the pistons further outwardly radially to thereby cause the powder to be ejected from the cavities into the appropriate receptacles.

It will be appreciated that whilst manually operable elements 37, 60 and 80 are shown in the embodiment described, these will normally be replaced by automatically operable devices such as motors to enable the device to operate automatically.

As an alternative to a foil having its ends clamped to the drum by the bolts 90 and clamp bar 92, the foil may be a welded ring of material.

The described embodiments use a single sheet of foil 14. In an alternative arrangement the foil 14 could include a number of segments 14a,b,c,d as shown in Figure 12. In radial cross-section the segments 14a,b,c,d may form arcs of a circle.

A fixed cam 29 is provided at one end of the drum 2. Each foil segment 14a,b,c,d is connected to a corresponding cam follower 27a,b,c,d that is rotatable relative to the cam 29. The cam 29 and cam followers 27a,b,c,d are arranged so that the powder compartments are open when the foil segments 14b,d are at the top and bottom positions (the 12 o'clock and the 6 o'clock positions). The powder compartments are arranged to be closed when they are away from these positions.

In one configuration the entire apparatus may be mounted on a carriage that can oscillate with a translational movement above a moving conveyor belt including powder receptacles. In this way the movement of the carriage can match movement of the receptacles so that the receptacles can be filled without any relative movement between them and the dispensing powder compartments in the drum. This arrangement may be desirable in order to optimise powder transfer into the receptacles.

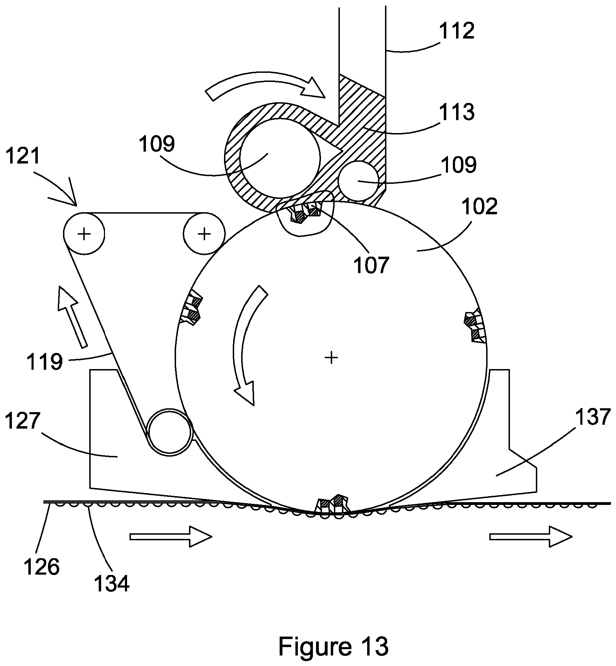

Figure 13 is a schematic view of another embodiment of a powder dispensing apparatus. The apparatus includes a drum 102 including a plurality of compartments 107 for accepting powder. A piston 124 defines the base of each compartment 107, as will be explained in more detail later. A hopper 112 is mounted above the drum assembly 102, and powder recirculating elements 109 are provided to aerate the powder 113 in the hopper 112.

A roller (not shown) is provided in the hopper 112 for filling compartments 107 with powder. The drum 102 is rotatable so that the compartments can rotate from the '12 o'clock' position to the '6 o'clock' position where the powder can be dispensed.

The apparatus includes a compaction station 121 where powder in the compartments 107 can be compressed. The compaction station 121 includes an endless belt 119 that is arranged to follow the motion of a compartment 107 along an arc of the drum 102. The powder in each compartment 107 can be compressed against the belt 119 by the piston 124. In this way the piston 124 can control the density or degree of compaction of powder in each compartment 107.

The apparatus also includes a first cleaning station 127 for the endless belt 119. The first cleaning station 127 is arranged to remove any excess powder from the endless belt 119. The first cleaning station 127 is located between the drum 102 and a conveyor belt 126 that carries a plurality of receptacles 134. Thus, the first cleaning station 127 can provide a cover for the incoming receptacles 134 to prevent any contamination before they are filled with powder.

The receptacles 134 are filled with powder when the compartments 107 are at the dispensing position. This is achieved by actuating the pistons 124, as described previously.

The apparatus also includes a second cleaning station 137 that is arranged to clean the compartments 107 and the pistons 124 once the dose has been dispensed. The second cleaning station is located between the drum 102 and the conveyor belt 126 so that it can provide a cover for the filled receptacles 134 and prevent any contamination with foreign objects or excess powder.

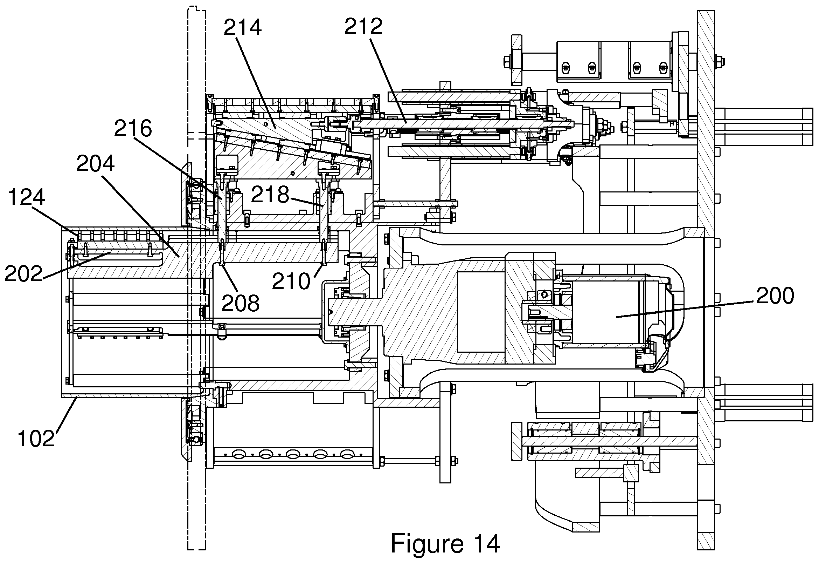

A cross-sectional view of the powder dispensing apparatus is shown in Figure 14. As is apparent from Figure 14, the drum 102 is connected to the remainder of the apparatus, which includes the driving mechanism, from one side only. In other words, the drum 102 is cantilevered with respect to the remainder of the apparatus.

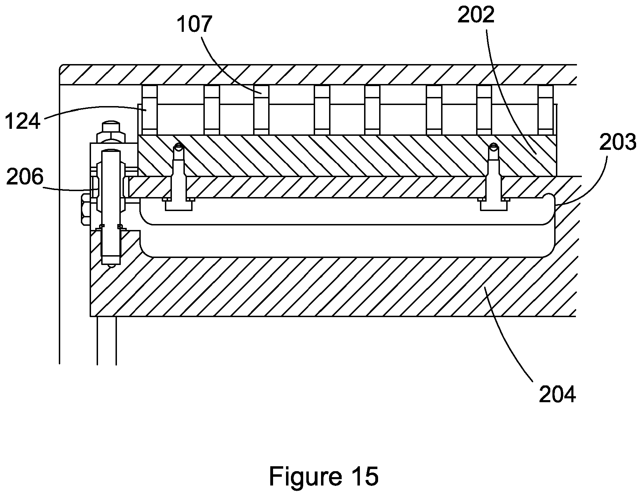

The drum 102 includes a plurality of pins 124 that act as pistons, extending radially from a piston plate 202, as can be seen in greater detail in Figure 15. The piston plate 202 is flexible relative to a main arm 204 that extends axially towards the drive mechanism. An adjustment screw 206 is provided in order to flex the piston plate 202 about a hinge point 203 relative to the main arm 204. The purpose of the adjustment screw 206 is to allow fine control of the orientation of the pins or pistons 124 relative to corresponding apertures in the drum 102. The piston plate 202 is flexed so that all of the pistons 124 can line up accurately with their corresponding aperture. This ensures that the pistons 124 can slide easily into the apertures so that no powder is lost around the edges of the pistons 124.

The pistons, the main arm 204 and the piston plate 202 can be connected to a drive mechanism so that they can be actuated. Bolts 208, 210 are provided in order to assemble the main arm 204 to the drive assembly.

The drive mechanism includes a main driving mechanism 212 that extends axially relative to the main axis of the drum 102. The main driving mechanism 212 is connected to a stroke reducer 214 which is a wedge-shaped member for converting axial motion into radial motion. The stroke reducer 214 is connected to two vertically extending members 216, 218 that are assembled to the main arm 204 with the bolts 208, 210. The stroke reducer 214 is arranged to convert axial motion into radial motion with a ratio of around 10:1. Thus the stroke reducer 214 provides a mechanism for enabling geared-down radial motion. This is advantageous because it allows very fine control of the position of the pistons 124.

The driving force that allows the pistons 124 to be actuated can be traced back to the main driving mechanism 212. The main driving mechanism 212 is located axially of the pistons 124 so that it is not a limiting factor when choosing a diameter for the drum 102. In this way it is possible to design a drum 102 with a reduced diameter because the drum 102 does not need to include a radial piston cylinder or spring.

The driving mechanism includes a motor 200 that is arranged to rotate the drum 102.

In order to remove the drum 102 from the drive assembly it is necessary merely to remove the bolts 208, 210. This allows the entire drum assembly, including the pistons 124 and their associated parts to be removed from the drive assembly. The drum assembly can then be cleaned in a remote location without exposing any of the drive assembly to cleaning products.

It will be appreciated that the present arrangement allows the drum 102 to be cantilevered relative to the drive assembly. In other words, the drum 102 is supported entirely from one side. This is advantageous because it facilitates easy removal of the drum 102 without completely stripping down the apparatus.

## Claims

1. An apparatus for dispensing a predetermined dose of powder, comprising:
a drum assembly (2; 102) having a main axis and at least one aperture (20) into which powder can be delivered at a filling position;
a piston (24; 124) associated with the or each aperture for dispensing powder out of the or each aperture at a dispensing position; and
a driving assembly (212) that is connectable to the piston, wherein the driving assembly is arranged to drive the piston radially with respect to the main axis of the drum assembly;
wherein the drum assembly (2; 102) is rotatable from the filling position to a dispensing position,
the driving assembly (212) is located axially of the piston with respect to the main axis of the drum assembly, and
the piston defines the base of a compartment (107) into which powder can be delivered and the position of the piston can be controlled in order to adjust the volume of the compartment,
**characterised in that**:
the drum assembly (102) includes a plurality of pins (24, 124) that act as the pistons, extending radially from a piston plate (202);
the piston plate (202) is flexible relative to a main arm (204) that extends axially towards the drive assembly (212); and an an alignment adjuster (206) arranged to adjust the inclination of the or each piston relative to its associated aperture by flexing the piston plate (202) so that all of the pistons (124) car line up accurately with their corresponding aperture.

2. Apparatus according to claim 1 comprising a plurality of pistons, each associated with a separate aperture.

3. Apparatus according to any of the preceding claims wherein the radial movement of the or each piston (24; 124) is provided by the geared-down motion of a component in the drive assembly.

4. Apparatus according to claim 3 wherein the piston is arranged to compress the powder in the compartment.

5. Apparatus according to claim 4 further including a compaction band (119) against which the piston can compress the powder, wherein the compaction band is arranged to follow the motion of the compartment, at least in part, while it moves from the receiving position to the dispensing position.

6. Apparatus according to any of the preceding claims wherein the drum assembly, the piston and the driving assembly are mounted on a carriage that permits translation of the components so that powder can be dispensed by the pistons while the drum assembly is translating.

7. Apparatus according to any of the preceding claims wherein the drum assembly is supported by the drive assembly from one side only.

8. Apparatus according to any of the preceding claims comprising a foil (14) having at least one through aperture (25), wherein the foil is moveable relative to the aperture (20) in the drum assembly, wherein the aperture (20) in the drum assembly can be aligned with the through aperture of the foil by said movement.

9. Apparatus according to claim 8 wherein the foil can be moved so that the aperture (20) in the drum assembly is misaligned with the through aperture (25) in the foil between the filling position and the dispensing position so that the compartment can be closed.

10. Apparatus according to any preceding claim further including a cleaning station arranged to clean the support portion, wherein the cleaning station is located between the dispensing position and the filling position.

## Patentansprüche

1. Vorrichtung zum Ausgeben einer vorgegebenen Dosis von Pulver, umfassend:
eine Trommelanordnung (2; 102) mit einer Hauptachse und mindestens einer Öffnung (20), in die Pulver an einer Füllposition zugeführt werden kann;
einen mit der oder jeder Öffnung assoziierten Kolben (24; 124) zum Ausgeben von Pulver aus der oder jeder Öffnung an einer Ausgabeposition; und
eine Antriebsanordnung (212), die mit dem Kolben verbunden werden kann, wobei die Antriebsanordnung dazu angeordnet ist, den Kolben in Bezug auf die Hauptachse der Trommelanordnung radial anzutreiben;
wobei die Trommelanordnung (2; 102) von der Füllposition zu einer Ausgabeposition gedreht werden kann,
wobei sich die Antriebsanordnung (212) in Bezug auf die Hauptachse der Trommelanordnung axial des Kolbens befindet, und
wobei der Kolben den Boden eines Raums (107) definiert, in den Pulver zugeführt werden kann, und die Stellung des Kolbens gesteuert werden kann, um das Volumen des Raums einzustellen,
**dadurch gekennzeichnet, dass**:
die Trommelanordnung (102) eine Vielzahl von Zapfen (24, 124) umfasst, die als die Kolben wirken und sich radial von einer Kolbenplatte (202) erstrecken;
die Kolbenplatte (202) relativ zu einem Hauptarm (204), der sich axial in Richtung der Antriebsanordnung (212) erstreckt, biegsam ist; und
eine Ausrichtungs-Einstelleinrichtung (206), die dazu angeordnet ist, die Neigung des oder jedes Kolbens relativ zu seiner assoziierten Öffnung durch Biegen der Kolbenplatte (202) derart einzustellen, dass alle Kolben (124) genau mit ihrer entsprechenden Öffnung fluchten können.

2. Vorrichtung nach Anspruch 1, umfassend eine Vielzahl von Kolben, die jeweils mit einer separaten Öffnung assoziiert sind.

3. Vorrichtung nach einem der vorangehenden Ansprüche, wobei die Radialbewegung des oder jedes Kolbens (24; 124) durch die untersetzte Bewegung einer Komponente in der Antriebsanordnung bereitgestellt wird.

4. Vorrichtung nach Anspruch 3, wobei der Kolben dazu angepasst ist, das Pulver in dem Raum zu verdichten.

5. Vorrichtung nach Anspruch 4, ferner umfassend ein Kompaktierungsband (119), an dem der Kolben das Pulver verdichten kann, wobei das Kompaktierungsband dazu angeordnet ist, der Bewegung des Raums mindestens teilweise zu folgen, während er sich von der Aufnahmeposition zu der Ausgabeposition bewegt.

6. Vorrichtung nach einem der vorangehenden Ansprüche, wobei die Trommelanordnung, der Kolben und die Antriebsanordnung auf einem Schlitten montiert sind, der die Verschiebung der Komponenten ermöglicht, sodass Pulver durch die Kolben ausgegeben werden kann, während die Trommelanordnung verschoben wird.

7. Vorrichtung nach einem der vorangehenden Ansprüche, wobei die Trommelanordnung nur von einer Seite aus von der Antriebsanordnung gestützt wird.

8. Vorrichtung nach einem der vorangehenden Ansprüche, umfassend eine Folie (14) mit mindestens einer Durchgangsöffnung (25), wobei die Folie relativ zu der Öffnung (20) in der Trommelanordnung beweglich ist, wobei die Öffnung (20) in der Trommelanordnung durch diese Bewegung auf die Durchgangsöffnung der Folie ausgerichtet werden kann.

9. Vorrichtung nach Anspruch 8, wobei die Folie derart bewegt werden kann, dass die Öffnung (20) in der Trommelanordnung zwischen der Füllposition und der Ausgabeposition nicht auf die Durchgangsöffnung (25) in der Folie ausgerichtet ist, sodass der Raum verschlossen werden kann.

10. Vorrichtung nach einem der vorangehenden Ansprüche, ferner umfassend eine Reinigungsstation, die dazu angeordnet ist, den Stützabschnitt zu reinigen, wobei sich die Reinigungsstation zwischen der Ausgabeposition und der Füllposition befindet.

## Revendications

1. Appareil servant à distribuer une dose prédéterminée de poudre, comportant :
un ensemble formant barillet (2 ; 102) ayant un axe principal et au moins une ouverture (20) dans laquelle de la poudre peut être délivrée au niveau d'une station de remplissage ;
un piston (24 ; 124) associé à la ou à chaque ouverture à des fins de distribution de poudre hors de la ou de chaque ouverture au niveau d'une position de distribution ; et
un ensemble d'entraînement (212) qui est en mesure d'être raccordé au piston, dans lequel l'ensemble d'entraînement est agencé pour entraîner le piston dans le sens radial par rapport à l'axe principal de l'ensemble formant barillet ;
dans lequel l'ensemble formant barillet (2 ; 102) est en mesure de tourner depuis la position de remplissage jusqu'à une position de distribution,
l'ensemble d'entraînement (212) est situé dans le sens axial du piston par rapport à l'axe principal de l'ensemble formant barillet, et
le piston définit la base d'un compartiment (107) dans lequel de la poudre peut être délivrée et la position du piston peut être commandée afin d'ajuster le volume du compartiment,
**caractérisé en ce que** :
l'ensemble formant barillet (102) comprend une pluralité d'axes de piston (24, 124) qui tiennent lieu de pistons, s'étendant dans le sens radial depuis une plaque de piston (202) ;
la plaque de piston (202) est flexible par rapport à un bras principal (204) qui s'étend dans le sens axial vers l'ensemble d'entraînement (212) ; et
un dispositif d'ajustement d'alignement (206) agencé pour ajuster l'inclinaison du ou de chaque piston par rapport à son ouverture associée en faisant fléchir la plaque de piston (202) de telle sorte que tous les pistons (124) peuvent s'aligner avec précision sur leur ouverture correspondante.

2. Appareil selon la revendication 1, comportant une pluralité de pistons, chacun étant associé à une ouverture séparée.

3. Appareil selon l'une quelconque des revendications précédentes, dans lequel le mouvement radial du ou de chaque piston (24 ; 124) est mis en œuvre par le mouvement démultiplié d'un composant dans l'ensemble d'entraînement.

4. Appareil selon la revendication 3, dans lequel le piston est agencé pour comprimer la poudre dans le compartiment.

5. Appareil selon la revendication 4, comportant par ailleurs une bande de compactage (119) contre laquelle le piston peut comprimer la poudre, dans lequel la bande de compactage est agencée pour suivre le mouvement du compartiment, au moins en partie, lors de son déplacement depuis la position de réception jusqu'à la position de distribution.

6. Appareil selon l'une quelconque des revendications précédentes, dans lequel l'ensemble formant barillet, le piston et l'ensemble d'entraînement sont montés sur un chariot qui permet la translation des composants de telle sorte que la poudre peut être distribuée par les pistons lors du mouvement de translation de l'ensemble formant barillet.

7. Appareil selon l'une quelconque des revendications précédentes, dans lequel l'ensemble formant barillet est supporté par l'ensemble d'entraînement au niveau d'un côté uniquement.

8. Appareil selon l'une quelconque des revendications précédentes, comportant une feuille métallique (14) ayant au moins une ouverture traversante (25), dans lequel la feuille métallique est mobile par rapport à l'ouverture (20) dans l'ensemble formant barillet, dans lequel l'ouverture (20) dans l'ensemble formant barillet peut être alignée sur l'ouverture traversante de la feuille métallique par ledit mouvement.

9. Appareil selon la revendication 8, dans lequel la feuille métallique peut être déplacée de telle sorte que l'ouverture (20) dans l'ensemble formant barillet est mal alignée par rapport à l'ouverture traversante (25) dans la feuille métallique entre la position de remplissage et la position de distribution de telle sorte que le compartiment peut être fermé.

10. Appareil selon l'une quelconque des revendications précédentes, comprenant par ailleurs une station de nettoyage agencée pour nettoyer la partie de support, dans lequel la station de nettoyage est située entre la position de distribution et la position de remplissage.
